# EUROPÄISCHE PATENTSCHRIFT

(11) **EP 1 141 264 B1**
(45) Veröffentlichungstag und Bekanntmachung des Hinweises auf die Patenterteilung: **16.08.2006**
(21) Anmeldenummer: 99965530.1
(22) Anmeldetag: 21.12.1999
(51) Int. Cl.: C12N 13/00, C12N 15/87, C12M 3/00, C12M 1/42

(54) **MIKROSYSTEME ZUR ZELLPERMEATION UND ZELLFUSION**
MICROSYSTEM FOR CELL PERMEATION AND CELL FUSION
MICROSYSTEMES POUR LA PERMEATION CELLULAIRE ET LA FUSION CELLULAIRE

(30) Priorität: 22.12.1998 DE 19859459
(43) Veröffentlichungstag der Anmeldung: 10.10.2001
(73) Patentinhaber: Evotec Technologies GmbH, 40225 Düsseldorf (DE)
(72) Erfinder: MÜLLER, Torsten, D-12439 Berlin (DE); SCHNELLE, Thomas, D-10243 Berlin (DE); SHIRLEY, Stephen, Graham, Brandon, Warks CV8 3HW (GB); FUHR, Günter, D-13187 Berlin (DE); GRADL, Gabriele, D-10557 Berlin (DE)
(74) Vertreter: Hertz, Oliver
(86) Internationale Anmeldenummer: PCT/EP1999/010277
(87) Internationale Veröffentlichungsnummer: WO 2000/037628

(56) Entgegenhaltungen:
- EP-A- 0 338 667
- WO-A-91/11262
- WO-A-93/05166
- US-A- 4 578 167
- US-A- 4 894 343
- FIEDLER S ET AL: "DIELECTROPHORETIC SORTING OF PARTICLES AND CELLS IN A MICROSYSTEM" ANALYTICAL CHEMISTRY,US,AMERICAN CHEMICAL SOCIETY. COLUMBUS, Bd. 70, Nr. 9, 1. Mai 1998 (1998-05-01), Seiten 1909-1915, XP000755524 ISSN: 0003-2700
- LUNDQVIST, J.A. ET AL.: "Altering the biological state of individual cultured cells and organelles with ultramicroelectrodes." PROC.NAT'L.ACD.SCI.USA, Bd. 95, September 1998 (1998-09), Seiten 10356-60, XP002134050

## Beschreibung

Die Erfindung betrifft eine Vorrichtung zur Manipulierung und Bearbeitung biologischer Objekte mittels elektrischer Pulse, insbesondere zur Permeation (Poration) und/oder Fusion von Zellen oder von synthetischen, membranumhüllten Gebilden wie Liposomen oder Vesikeln, oder zur Permeation von Membran- oder Schichtmaterialien in miniaturisierten Elektrodenstrukturen, und Manipulierungs- oder Bearbeitungsverfahren unter Verwendung einer derartigen Vorrichtung.

Für bestimmte biotechnologische, medizinische oder gentechnische Aufgaben ist die kurzzeitige und reversible Erhöhung der Durchlässigkeit der Umhüllung lebender, in einer Flüssigkeit suspendierter Zellen von Interesse (Übersicht in "Elektromanipulation of Cells", U. Zimmermann, G.A. Neil, CRC, 1996). Neben chemischen, virusbasierten und laserinduzierten Permeationsmethoden hat sich wegen der Einfachheit und Definiertheit der Applikation die Permation mittels kurzer elektrischer Pulse (Elektroporation oder Elektropermeation) entwickelt, wobei gepulste Gleichstromsignale (s. U. Zimmermann et al. in "BBA", Band 641, 1982, S. 160 ff.) oder gechopperte HF-Felder (s. PCT/US88/03457) verwendet werden. Bei den bisher allgemein bekannten, kommerziell verfügbaren Elektroporationsgeräten werden die Elektroporationen und/oder Fusionen in Kammern mit Elektroden durchgeführt, deren Dimensionen wesentlich größer als die Dimensionen der behandelten Objekte sind, wobei die folgenden Nachteile auftreten.

Zellen können bisher nicht in Kulturmedien permeiert werden, da diese eine hohe Leitfähigkeit besitzen und aufgrund der niedrigen Dielektrizitätskonstante und Leitfähigkeit biologischer Zellen das elektrische Feld außerhalb der Zellen verlaufen würde. Außerdem würden die Kulturmedien zu einer hohen thermischen Belastung der zu behandelnden Zellen durch Widerstandsheizen aufgrund des Stromflusses durch das Kulturmedium führen. Der Anwendungsbereich der herkömmlichen Elektroporationsgeräte ist ferner auf robuste und widerstandsfähige Zellen beschränkt. Außerdem ist eine Optimierung der Fusionsparameter wie z.B. der Feldstärke und der Pulsfrequenz nur eingeschränkt möglich. Dies ergibt sich aus der Größenabhängigkeit des maximal induzierten Transmembranpotentials V_{M}^{max} einer sich in einem externen elektrischen Feld E befindlichen Zelle (Radius R) gemäß V_{M}^{max} = 1,5 · R · E und der praktischen Größenvarianz biologischer Objekte. Dies ist insbesondere dann problematisch, wenn unterschiedliche Zelltypen gleichzeitig permeiert bzw. fusioniert werden sollen oder nur wenige Ausgangszellen zur Verfügung stehen. Schließlich erlauben die herkömmlichen Elektroporationsgeräte keine zuverlässige Einzelzellmanipulation oder -permeation.

Es ist ferner allgemein bekannt, daß sich biologische Objekte auf der Grundlage negativer oder positiver Dielektrophorese mit hochfrequenten elektrischen Feldern manipulieren lassen. Dies wird insbesondere in Mikrosystemen realisiert, wie es beispielsweise von G. Fuhr et al. in "Naturwissenschaften", Band 81, 1994, Seiten 528 ff. beschrieben ist. So zeigen bei einer üblichen Lösungsmittelleitfähigkeit von rund 0.3 S/m biologische Zellen unter der Wirkung hochfrequenter elektrischer Felder über einen großen Frequenzbereich von rd. 1 MHz bis über 120 MHz negative Dielektrophorese, d.h. die Zellen werden von mit den Hochfrequenzfeldern beaufschlagten Elektroden zu Gebieten niedriger Feldstärke bewegt. In Kulturmedien mit Leitfähigkeiten über 1 S/m zeigen tierische Zellen über alle Frequenzen negative Dielektrophorese.

Aus JP 60-251876 ist eine Einrichtung zur Zellfusion bekannt, bei der biologische Zellen unter der Wirkung elektrophoretiischer Kräfte zwischen planaren Elektroden angeordnet und mittels Hochspannungsbehandlung fusioniert werden. Die Elektroden befinden sich an den Kanalwänden eines Mikrosystems. Zur Fusion werden die Zellen auf den Elektrodenoberflächen festgesetzt. Das Mikrosystem besitzt derart geringe Dimensionen, daß sich die auf gegenüberliegenden Elektroden angeordneten Zellen gegenseitig berühren. Diese Fusionstechnik besitzt mehrere Nachteile. Die Positionierung der Zellen auf den Elektroden und ihre rückstandsfreie Entfernung nach der Fusion sind schwierig und zeitaufwendig. Eine bestimmte Kanalstruktur kann immer nur für eine bestimmte Zellgröße verwendet werden. Die Fusion ist nicht reproduzierbar, da sich zwischen den Elektroden gegebenenfalls mehrere Zellen ansammeln.

Aus JP 63-152971 ist ein Durchflußsystem zur Zellfusion und zur Nukleinsäureübertragung bekannt, bei dem an zwei Wänden einer Durchflußkammer Elektrodenplatten angebracht sind. Die Elektrodenplatten können anwendungsabhängig mit Gleich- und Wechselspannungen beaufschlagt werden, um Zellen, die durch die Durchflußkammer gespült werden, einer elektrischen Behandlung zu unterziehen. Die Durchflußkammer dieses Systems ist manuell demontierbar. Sie besitzt eine Größe, die erheblich größer als die zu behandelnden Zellen ist, und damit die gleichen Nachteile wie die obengenannten herkömmlichen Elektroporationsgeräte mit geschlossenen Kammern (ohne Durchfluß). Im Durchflußsystem ergibt sich ein weiterer Nachteil durch die unreproduzierbare, undefinierte Position der zu behandelnden Objekte. Dementsprechend können auch keine reproduzierbaren Fusionsergebnisse erzielt werden.

Die Sortierung biologischer Zellen mittels negativer Dielektrophorese wird auch in der Publikation von S. Fiedler et al. in "Analytical Chemistry", Band 70, 1998, S. 1909-1915 beschrieben.

In US 4 578 167 und EP 338 667 A1 sind Vorrichtungen zur Elektrofusion biologischer Zellen in Elektrodenkammern mit Querschnittsdimensionen im mm- bis cm-Bereich beschrieben.

Es ist die Aufgabe der Erfindung, eine verbesserte Vorrichtung zur Manipulierung oder Bearbeitung (insbesondere durch Permation) mikroskopischer Objekte zu schaffen, deren Anwendungsbereich in Bezug auf die Auswahl der Umgebungs- oder Kulturmedien, die Optimierung der Porationsparameter und/oder die Handhabung kleinster Objektmengen (bis hin zu Einzelobjekten) erweitert ist. Die Erfindung soll insbesondere ermöglichen, eine reproduzierbare Manipulierung bzw. Bearbeitung, z.B. entsprechend einem definierten Protokoll und gegebenenfalls unter Gewährleistung einer freien Beobachtbarkeit, durchzuführen. Die Aufgabe der Erfindung besteht auch darin, ein verbessertes Elektroporationsverfahren unter Verwendung einer derartigen Vorrichtung anzugeben.

Diese Aufgaben werden durch eine Vorrichtung, ein Elektroporationsgerät bzw. ein Verfahren mit den Merkmalen entsprechend den Patentansprüchen 1, 7, 8 bzw. 10 gelöst. Vorteilhafte Ausführungsformen der Erfindung ergeben sich aus den abhängigen Ansprüchen.

Die Grundidee der Erfindung besteht darin, von den herkömmlichen, makroskopischen Elektroporationsanordnungen zu Mikrosystemen überzugehen, bei denen die Objektbehandlung als Behandlung frei suspendierter Teilchen in Umgebungs- oder Kulturmedien zwischen miniaturisierten Elektroden stattfindet. Gemäß einem ersten wichtigen Aspekt der Erfindung sind die Elektroden in einem Mikrosystem mit einer Kanalstruktur vorgesehen. Im Unterschied zur herkömmlichen Elektroporation in geschlossenen Küvetten ist die Kanalstruktur als Durchflußsystem eingerichtet. Die zu behandelnden Objekte werden somit vom strömenden oder fließenden Medium zu den Elektroden geführt und beim Durchfluß oder während eines zeitlich begrenzten, ortsfesten dielektrophoretischen Positionierens der Objekte in Bezug auf die Elektroden permeiert. Während der elektrischen Behandlung der Objekte besitzen diese in der freien Suspension einen Abstand von den Elektroden. Die Behandlung erfolgt in Bezug auf die Elektroden und/oder Wände des Mikrosystems berührungsfrei. Gemäß einem zweiten wichtigen Aspekt der Erfindung werden die Objekte mit einem derart geringen Abstand von den Elektroden permeiert, daß selbst in hochleitfähigen Medien eine Permeation erfolgen kann. Im Mikrosystem sind Elektroden zur Ausübung von Polarisationskräften auf der Grundlage negativer Dielektrophorese und Elektroden zur Elektroporation vorgesehen.

Gemäß einem dritten wichtigen Aspekt der Erfindung sind Elektroden vorgesehen, die eine Doppelfunktion erfüllen. Eine erfindungsgemäße Vorrichtung weist z.B. ein Elektrodensystem auf, das simultan zur Halterung der Objekte im Medium oder zur Führung der Objekte in einem strömenden Medium sowie zur Beaufschlagung der Objekte mit elektrischen Feldern zur Realisierung der Elektroporation eingerichtet ist. Im Unterschied zu herkömmlichen Elektroporationsgeräten bilden die Elektroden erfindungsgemäß einen mindestens in zwei zueinander senkrechten Raumrichtungen geschlossenen Käfig, in dem die Objekte manipuliert und der Elektroporation ausgesetzt werden. Die Elektroden sind zur Erzeugung eines inhomogenen elektrischen Feldes im Kanal eingerichtet, das ein sich in Strömungsrichtung lang erstreckendes Minimum aufweist. Die zu behandelnden Objekte werden mit den sich in Kanalrichtung durchgehend oder mit Unterbrechungen angeordneten Elektroden, die gleichzeitig Fokussierungs- und Porationselektroden sind, im Feldminimum gehalten.

Gemäß einer bevorzugten Ausführungsform der Erfindung ist das Elektrodensystem so eingerichtet, daß die Poration der Objekte entsprechend vorbestimmter Porationsmuster erfolgt. Hierzu besitzen die Elektroden (Pulselektroden) feldformende Einrichtungen wie z.B. Elektrodenspitzen, die entsprechend den gewünschten Porationsmustern angeordnet sind, oder Abschirm- oder Abdeckelemente, die ein Freiliegen der Elektroden in Bezug auf das Medium mit dem oder den Objekten entsprechend dem gewünschten Porationsmuster erlauben.

Eine erfindungsgemäße Vorrichtung wird vorzugsweise als Mikrosystem mit Kanalstrukturen aufgebaut, die in mindestens einem Bereich mit einem erfindungsgemäßen Elektrodensystem ausgestattet sind (Elektroporationsbereich). Vorteilhafterweise werden derartige Elektroporationsbereiche im Mikrosystem mit anderen Bereichen zur Behandlung oder Manipulierung der Objekte z.B. zum Sammeln oder Trennen bestimmter Objekttypen (Manipulierungsbereich) kombiniert. Erfindungsgemäße Mikrosysteme werden vorzugsweise als Durchflußsysteme betrieben.

Die Erfindung besitzt die folgenden Vorteile. Erfindungsgemäße Vorrichtungen erlauben Objektpermeationen in physiologischen Lösungen. Somit wird die Anwendbarkeit der Elektroporation auf Medien mit hoher Leitfähigkeit (z.B. im Bereich von 0.01 bis 10 S/m) erweitert. Es wird erstmalig eine zuverlässige, berührungsfreie und schonende Permeation einzelner Objekte oder Objektgruppen in freier Lösung ermöglicht. Die Miniaturisierbarkeit des Systems erlaubt eine Erhöhung der Elektrodenhaltbarkeit und eine Verringerung der Elektroporations-Pulsamplituden (bis in den Volt- bis 100V-Bereich), wobei dennoch die erforderlichen hohen Feldstärken erzielbar sind. Es wird erstmals ermöglicht, die zu behandelnden Objekte simultan an mehreren Stellen entsprechend vorbestimmten definierten Porationsmustern zu behandeln. Es wird eine Kombination der bisher auf makroskopische Anwendungen beschränkten Elektroporationstechnik mit Verfahrensweisen der Mikrosystemtechnik ermöglicht. Die erfindungsgemäße Objektbehandlung erfolgt berührungslos. Beschränkungen in Bezug auf die Anpassung des Mikrosystems an eine bestimmte Objektgröße sind ausgeschlossen. Außerdem erfolgt die Objektbehandlung rückstandsfrei. Verunreinigungen der Elektroden werden vermieden.

Weitere Vorteile bestehen in der erhöhten Effizienz und Ausbeute der Elektroporation, der verminderten Wärmeproduktion durch Minimierung der Elektrodenoberfläche, der Möglichkeit der Permeation unterschiedlich großer Objekte und der zeiteffektiven Permeation von Objekten in Durchflußsystemen bei niedrigen Spannungen.

Die Erfindung ist nicht auf biologische Zellen beschränkt, sondern kann entsprechend mit allen interessierenden synthetischen, membranumhüllten Gebilden wie Liposomen oder Vesikeln oder mit Membran- oder Schichtmaterialien implementiert werden.

Weitere Vorteile und Einzelheiten der Erfindung werden im folgenden unter Bezug auf die beigefügten Zeichnungen erläutert. Es zeigen:
- Fign. 1A, 1B: zwei Ausführungsformen von Elektrodensystemen in erfindungsgemäßen Vorrichtungen in schematischer Perspektivansicht,
- Fign. 2A, 2B: zwei weitere Ausführungsformen von Elektrodensystemen in schematischer Perspektivansicht,
- Fig. 3: eine weitere Ausführungsform eines Elektrodensystems mit planaren, strukturierten Elektroden,
- Fig. 4, 5: schematische Darstellungen zur Illustration des Feldlinienverlaufs bei der Elektroporation,
- Fig. 6: eine schematische Perspektivansicht zur Illustration erfindungsgemäßer feldformender Einrichtungen am Elektrodensystem,
- Fig. 7: eine schematische Darstellung eines Mehrkanal-Mikrosystems, das zur erfindungsgemäßen Poration von Objekten unterschiedlicher Größe eingerichtet ist,
- Fig. 8: eine schematische Darstellung eines erfindungsgemäßen Elektroporators mit Durchflußbetrieb,
- Fig. 9: schematische Darstellungen weiterer Elektrodenformen für dreidimensionale Anordnungen von Elektrodensystemen,
- Fig. 10: schematische Darstellungen zur Illustration der Kombination von Porations- und Manipulierungsbereichen in erfindungsgemäßen Mikrosystemen, und
- Fig. 11: eine weitere Ausführungsform eines Elektrodensystemen in schematischer Draufsicht,

Die folgende Erläuterung bezieht sich insbesondere auf die Elektrodengestaltung und -beschaltung in erfindungsgemäßen Vorrichtungen. Einzelheiten der Herstellung von Mikrosystemen mit halbleitertechnologischen Mitteln, der Kombination von Mikrosystemen mit Probenzufuhr- oder Detektionssystemen, der Erzeugung der Hochfrequenzfelder zur Dielektrophorese und der Gestaltung der Permeationspulse werden nicht im einzelnen erläutert, soweit diese an sich aus den herkömmlichen Techniken bekannt sind und in analoger Weise bei der erfindungsgemäßen Vorrichtung übernommen werden können. Bei den folgenden Darstellungen wird in der Regel ein rundes Objekt gezeigt, das stellvertretend für die obengenannten, zu behandelnden Objekte dargestellt wird. Der Schwerpunkt der folgenden Erläuterung liegt bei der Elektroporation in Durchflußsystemen. Hierbei wird der Elektroporationsbereich der erfindungsgemäßen Vorrichtung von einem Medium (Umhüllungs- oder Kulturmedium) mit den zu behandelnden Objekten durchströmt, wobei miniaturisierte Elektroden Feldbarrieren in Richtungen senkrecht zur Strömungsrichtung (zweidimensionale Feldkäfige) bilden. Die Erfindung ist entsprechend in stationären Systemen insbesondere mit dreidimensionalen Feldkäfigen implementierbar.

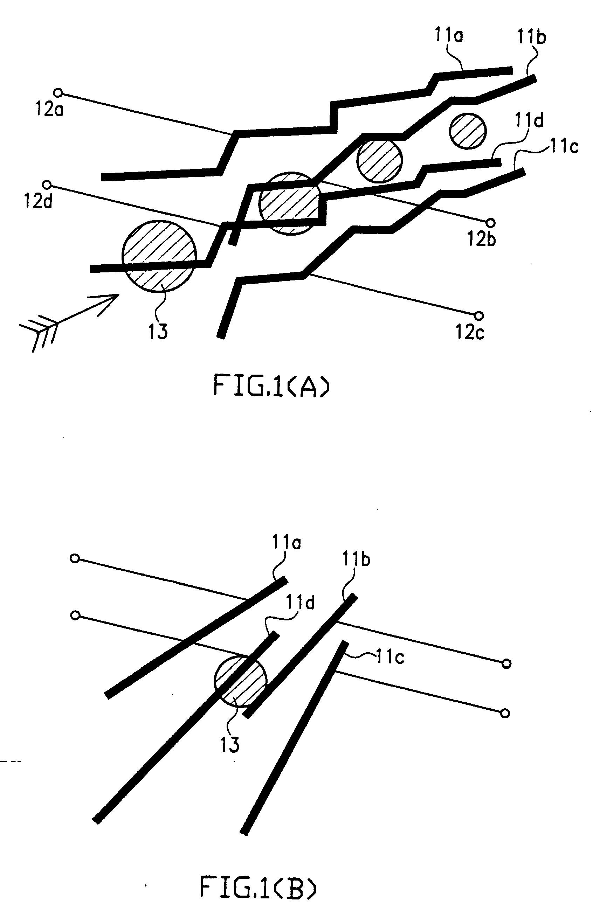

Die Fign. 1A, 1B zeigen beispielhafte Ausführungsformen von Elektrodensystemen von Durchflußanordnungen erfindungsgemäßer Vorrichtungen. In Mikrosystemen mit Kanalstrukturen (beispielsweise halbleitertechnologisch prozessierte Kanäle in Si-Chips) sind jeweils zwei der zum Elektrodensystem gehörenden vier Elektroden an der oberen bzw. unteren Kanalbegrenzung (Deckel bzw. Boden) angebracht. Die perspektivischen Darstellungen zeigen die Elektroden 11a und 11b als obere Elektroden und die Elektroden 11c und 11d als untere Elektroden. Die nicht dargestellten Kanalbegrenzungen sind vorzugsweise ebene Halbleiteroberflächen im Chip, auf denen die Elektroden mit geeigneten Depositionsverfahren gebildet sind. Es ist hervorzuheben, daß die Elektroden im wesentlichen schichtförmig auf den Kanalbegrenzungen aufgebracht sind und somit nicht bzw. nur geringfügig in den Kanal hineinragen. Gesonderte seitliche Kanalbegrenzungen zwischen den oberen Elektroden einerseits und den unteren Elektroden andererseits sind aufgrund der Feldkäfigfunktion des Elektrodensystems nicht erforderlich. Das Elektrodensystem ist zur Ausbildung elektrischer Feldbarrieren auf der Grundlage negativer Dielektrophorese zwischen den Objekten 13 und den oberen bzw. unteren Kanalbegrenzungen bzw. den sich seitlich anschließenden Kanalräumen eingerichtet. Die Feldbarrieren bilden zusammen ein Feldminimum, das sich in Kanallängsrichtung erstreckt.

Im Unterschied zu herkömmlichen Elektroporationskammern werden erfindungsgemäß mindestens zwei (vorzugsweise vier oder mehr) Elektroden dreidimensional in einem Kanal angeordnet. Die Objekte 13 (z.B. biologische Zellen) werden durch eine Medienströmung oder eine andere Kraft (Pfeilrichtung in Fig. 1A) durch das Elektrodensystem geführt und auf der Grundlage negativer Dielektrophorese von den Elektroden weg in den Zentralbereich des Kanals gedrückt. Zur Erzielung bestimmter Bewegungsabläufe können die Elektroden 11a-11d bandartig verschieden gestaltet sein, so z.B. kurvenförmig (Fig. 1A) oder gerade (Fig. 1B).

Das Elektrodenmaterial wird anwendungsabhängig gewählt und besteht vorzugsweise aus Platin, Titan, Tantal oder Legierungen aus diesen. Die Dicke der Elektrodenbänder liegt im µm-Bereich und ist vorzugsweise < 1 µm. Charakteristische Kanalquerdimensionen liegen im Bereich von rd. 1 µm bis 100 µm.

Die Elektroden 11a-11d sind jeweils entsprechend mit elektrischen Steuerleitungen 12a-12d verbunden. Die Steuerleitungen führen zu einer (nicht dargestellten) Steuereinrichtung, die einen Hochfrequenzgenerator zur Erzeugung der Hochfrequenzfelder für die negative Dielektrophorese und einen Pulsgenerator zur Erzeugung der Permeationspulse enthält. In diesem Fall funktionieren die Elektroden simultan als Hochfrequenz- und Pulselektroden.

Beim erfindungsgemäßen Betrieb werden die zu behandelnden Objekte 13 mit dem Medium durch den Kanal mit dem Elektrodensystem 11a-11d geführt. Das Elektrodensystem wird simultan mit HF-Spannungen (Bereich z.B. 1 MHz bis über 120 MHz) und Permeationspulsen (Dauer µs-Bereich, Amplitude bis 100 V) beaufschlagt. Die Objekte werden somit in den Zentralbereich des Kanals gedrückt, wo die Permeationspulse wirken, die zu einer reversiblen Öffnung der Zellmembran führen. Die gleichzeitige Nutzung des Elektrodensystems für beide Funktionen stellt einen besorideren und bei der früheren Betrachtung herkömmlicher Elektroporationsgeräte und Mikrosysteme nicht erwarteten Vorteil dar. Es kann auch ein quasikontinuierlicher Durchflußbetrieb vorgesehen sein, bei dem eine Gruppe der zu behandelnden Objekte mit dem Suspensionsmedium in das Elektrodensystem geführt und dort unter Nutzung dielektrophoretischer Kräfte festgehalten wird. Anschließend erfolgt die Objektbehandlung (Elektroporation, Fusion oder dgl.) für alle Objekte unter definierten Bedingungen gleichzeitig. Danach werden die dielektrophoretischen Kräfte, die die Objekte im Elektrodensystem gehalten haben, freigegeben. Die Objekte strömen im Mikrosystem weiter.

Die in Kanalströmungsrichtung wirksame Länge der Elektroden 11a-11d kann anwendungsabhängig verschieden ausfallen bzw. dazu eingerichtet sein, daß mehrere Objekte 13 (Fig. 1A) gleichzeitig, aber räumlich getrennt im Elektrodensystem permeiert werden.

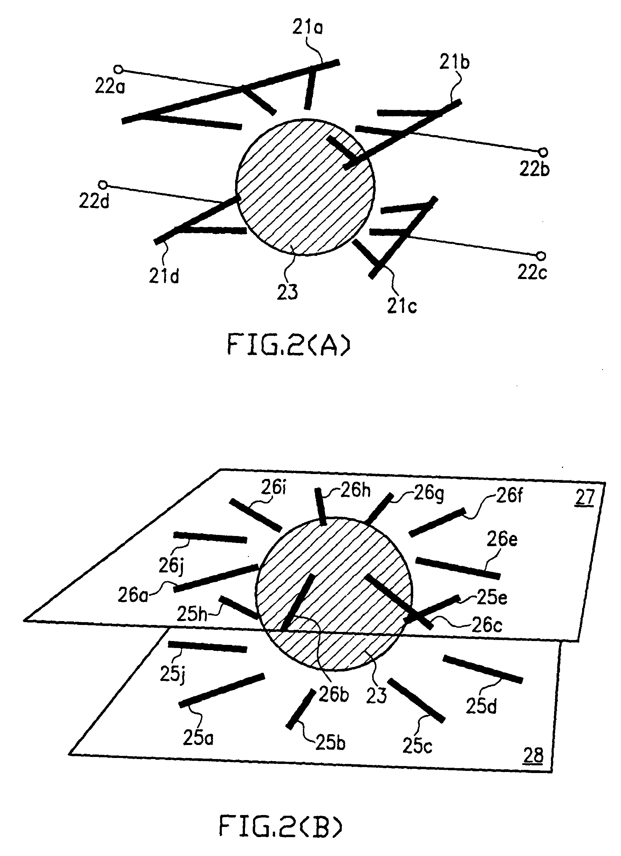

Ein besonderer Vorteil der Erfindung, nämlich die Bereitstellung vorbestimmter Porationsmuster, wird im folgenden unter Bezug auf die Fign. 2A, 2B erläutert, die zwei Ausführungsformen mit Elektrodensystemen zeigen, die mit feldformenden Einrichtungen versehen sind. Die Elektrodensysteme bestehen aus den Elektroden 21a-21d mit den entsprechenden elektrischen Steuerleitungen 22a-22d und feldformenden Elektrodenelementen 25 und 26. In Fig. 2A ist aus Übersichtlichkeitsgründen wie bei Fig. 1A, 1B nur das Elektrodensystem und das Objekt 23 ohne die Kanalbegrenzungen und ohne andere Teile des Mikrosystemschips dargestellt. In Fig. 2B sind lediglich die feldformenden Elemente (oder separaten Elektroden) 25a-25j, 26a-26j, das Objekt 23 und zur Verdeutlichung die Ebenen der oberen bzw. unteren Kanalbegrenzung 27, 28, nicht jedoch ggf. vorhandene, die Elemente in Gruppen verbindende Elektrodenbänder bzw. die elektrischen Steuerleitungen dargestellt.

Die feldformenden Elemente 25, 26 sind Elektrodenstukturen, die sich band- oder linienförmig auf der jeweiligen Kanalbegrenzungsoberfläche hin zur Kanalmitte erstrecken. Die Längsdimension hin zur Kanalmitte wird derart gewählt, daß die Enden der feldformenden Elemente nur noch einen Abstand von rd. einem bis einem halben Objektdurchmesser vom Objekt 23 entfernt sind. Die Querdimensionen werden so gewählt, daß sich spitzenförmige Strukturen ergeben, die wesentlich dünner als die charakteristische Objektdimension sind.

Die feldformenden Elemente 25, 26 verursachen eine hohe Feldlinienkonzentration und somit hohe Feldstärken in lokal eng begrenzten Bereichen. Damit ergibt sich auch im Medium eine Feldlinienbündelung und somit ein dielektrischer Durchschlag an bestimmten Orten auf der Oberfläche des Objekts 23. Die örtliche Verteilung der Durchschläge entspricht der Verteilung der feldformenden Elemente und ergibt ein vorbestimmtes Porationsmuster.

Die Fig. 2A zeigt eine Ausführungsform mit vier Elektroden 21a-21d mit einer räumlichen Anordnung analog zu Fig. 1A. Die Fig. 2B hingegen ist eine Multielektrodenanordnung, die als Durchflußsystem oder als stationäres System mit einem geschlossenen Feldkäfig betrieben werden kann. Es ist möglich, daß jedes der feldformenden Elemente 25a-25j, 26a-26j gemäß Fig. 2B eine separate Elektrode mit einer eigenen elektrischen Steuerleitung darstellt. Erfindungsgemäß ist es nicht zwingend erforderlich, daß die Elektroden die genannte Doppelfunktion erfüllen. Sind beispielsweise viele, fein strukturierte feldformende Elemente für die Elektroporation gemäß Fig. 2B vorgesehen, so ist es möglich, daß diese Spitzenelektroden bei Ansteuerung mit einer Hochfrequenz-Spannung keine genügenden Feldkräfte aufbringen, um das zu behandelnde Objekt im interessierenden Bereich zu positionieren oder zu fokussieren. In diesem Fall können gesonderte (nicht dargestellte) Elektroden zur Erzeugung geeigneter Feldbarrieren vorgesehen sein.

Zur Erhöhung der lokalen Feldlinienkonzentration können an den kanalseitigen Enden der feldformenden Elemente zusätzlich (nicht dargestellte) Spitzen- oder Kantenstrukturen vorgesehen sein.

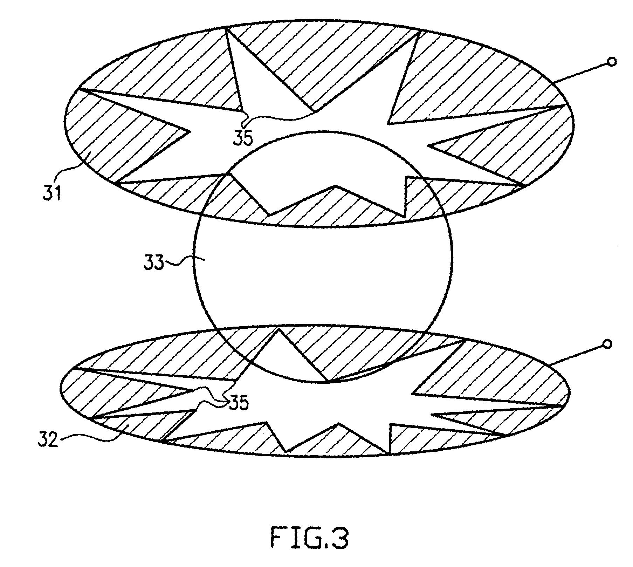

Fig. 3 zeigt eine weitere Ausführungsform mit planaren oder scheibenförmigen Elektroden 31, 32, zwischen denen das Objekt 33 mit einem Durchflußsystem hindurchgeführt wird. Diese Ausführungsform ist somit eine Zwei-Elektrodenanordnung, bei der wieder die Elektroden eine Doppelfunktion in Bezug auf die Dielektrophorese und die Elektroporation erfüllen. Jede der Elektroden 31, 32 besitzt eine innere sternförmige Ausnehmung, so daß das Elektrodenmaterial Spitzen 34 bildet, die analog zu den feldformenden Elementen 25, 26 in den Fign. 2A, 2B Feldlinienkonzentrationen und somit ein bestimmter Porationsmuster auf dem Objekt 33 ergeben, sobald die Durchschlagsspannung erreicht ist.

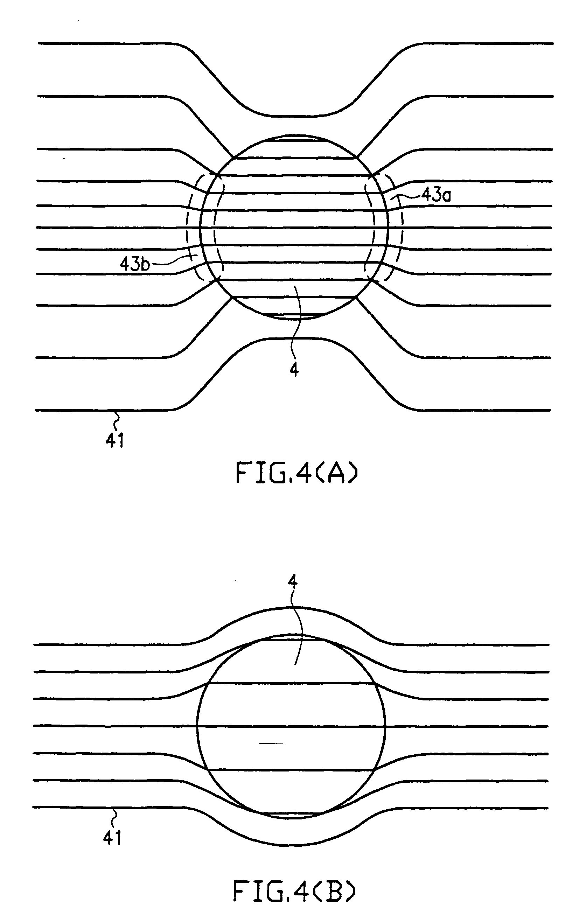

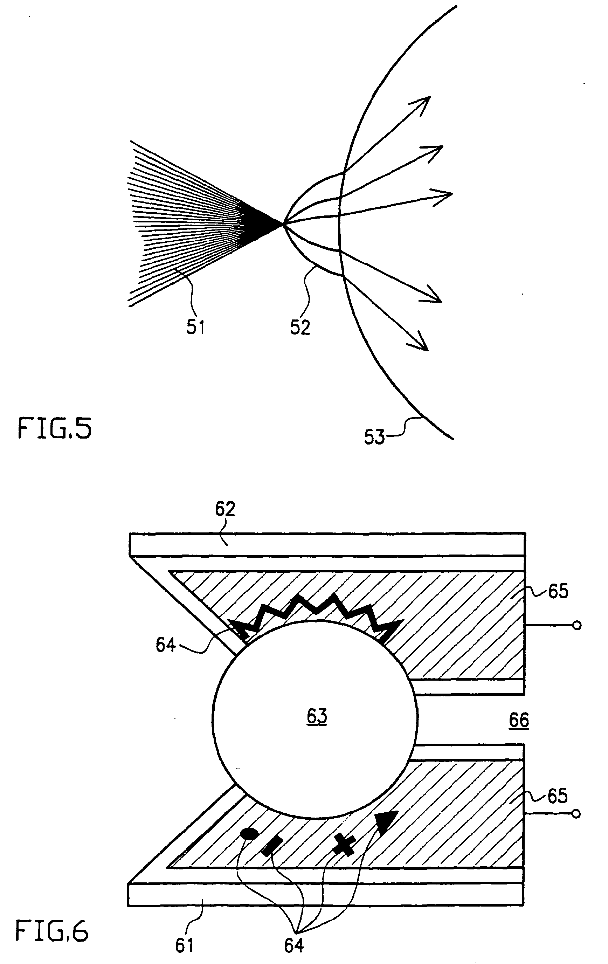

Die Fign. 4 und 5 illustrieren die feldformende Wirkung der Elektroden bzw. der feldformenden Elemente bei erfindungsgemäßen Vorrichtungen. Fig. 4a zeigt den Feldlinienverlauf in der Nähe und durch eine biologische Zelle 43 bei niedriger Außenleitfähigkeit, wie er beispielsweise bei herkömmlichen Permeationsanordnungen gegegen ist. Fig. 4b zeigt die Situation bei hoher Außenleitfähigkeit. Die bei niedriger Außenleitfähigkeit auftretende Bündelung der Feldlinien 41 im Bereich 43a, 43b der Oberfläche des Objekts 43 kann bei höher leitender Außenlösung nicht erreicht werden, da die Feldlinien 41 das Objekt 43 vorrangig umfließen und weniger durchsetzen. Wird hingegen gemäß Fig. 5 das Ende der Elektrode bzw. des feldformenden Elements 51 mit möglichst geringer Querdimension (d.h. möglichst spitz, Krümmungsradus << Objektradius) nahe an die Objektoberfläche (z.B. Oberfläche einer biologischen Zelle) 53 angeordnet, so können auch in hoch leitfähigen Außenlösungen (physiologische Kulturmedien) genügend hohe Feldstärken für die Elektroporation erzielt werden. Der Abstand der Spitze der Elektrode 51 von der Objektoberfläche beträgt vorzugsweise weniger als ein Objektdurchmesser. Die Feldlinien 52 zeigen den Bereich des dielektrischen Durchbruchs durch die Membran oder Oberfläche des Objekts 53.

Eine andere Gestaltung feldformender Elemente ist in Fig. 6 illustriert. Bei diesem Elektrodensystem sind zwei Elektroden 61, 62 in planarer, flächiger Gestaltung vorgesehen. Die feldformenden Elemente werden durch Isolationsschichten 65 auf jeder der Elektroden auf der dem Kanal bzw. den Objekten zugewandten Seiten gebildet. Die Isolationsschichten 65 besitzen an vorbestimmten Positionen Ausnehmungen oder Öffnungen 64, an denen die metallische Elektrodenfläche (dunkel dargstellt) mit dem Medium in Berührung kommt. Die Ausnehmungen 64 können in vorbestimmter Weise mit den verschiedensten Öffnungsformen mit eckigen, kurvenförmigen oder langgestreckten Umhüllungen vorgesehen sein. Die Elektroden 61, 62 sind auf Substraten (nicht dargestellt) mit den Methoden der Halbleitertechnologie prozessiert. Als Substratmaterial kommt hier wie bei den anderen Ausführungsformen neben Halbleitermaterial (z.B. Silizium) auch Glas, Kunststoff, Keramik oder dgl. in Frage. Das Bezugszeichen 63 bezeichnet das Objekt, das im Kanal zwischen den Elektroden 61, 62 hindurchgeführt oder hindurchgeströmt wird.

Die Ausnehmungen 64 bewirken analog zur Funktion der feldformenden Elemente in Spitzenform eine Konzentration der Feldlinien, so daß sich eine Permeation des Objekts 63 an bestimmten Stellen ergibt bzw. das vorbestimmte Porationsmuster ergibt.

Ein besonderer Vorteil der Ausführungsform gemäß Fig. 6 besteht darin, daß Belastungen des Elektrodenmaterials durch relativ hohe Pulsspannungen verringert werden. Die Pulsspannungen können hier wie bei den anderen Ausführungsformen im Bereich von 1 V bis zu einigen 100 V liegen.

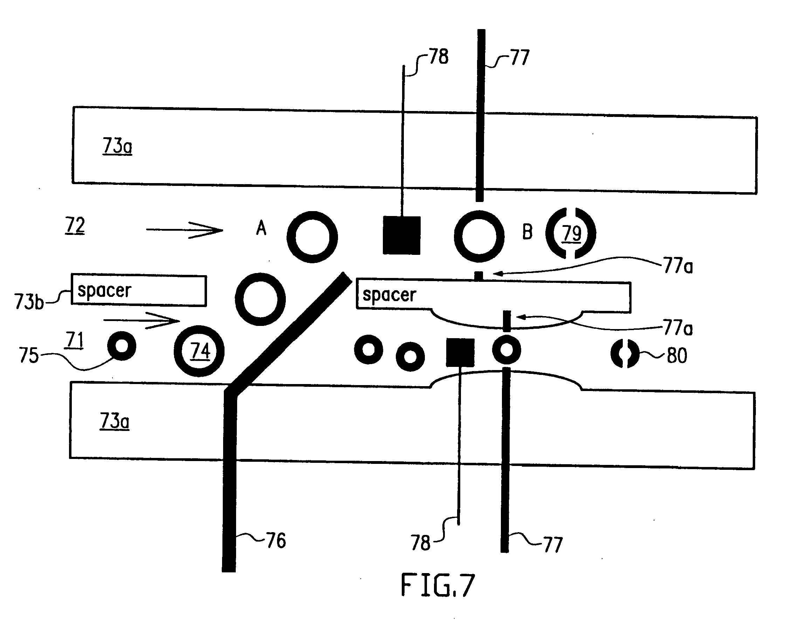

Gegenstand der Erfindung ist auch die Kombination eines miniaturisierten Elektroporationssystems gemäß einer der oben erläuterten Ausführungsformen mit einem miniaturisierten Objektmanipulator und/oder -detektor, wobei eine Elektroporation der Objekte nur an vorbestimmten Objekten oder entsprechend vorbestimmter Zeitmuster vorgenommen wird. Ein derartiges System kann anwendungsabhängig analog zu den in Fig. 7 gezeigten Grundstrukturen gestaltet sein.

Fig. 7 ist eine schematische Draufsicht auf ein miniaturisiertes Mehrkanalsystem, bei dem zwei Kanäle 71, 72 vorgesehen sind, die von Randelementen 73a begrenzt und durch Trennelemente 73b (Spacer) getrennt sind. Die Kanäle 71, 72 werden in Pfeilrichtung von einem Medium durchströmt.

Das Mikrosystem umfaßt einen Manipulationsbereich A und einen Elektroporationsbereich B. Im Manipulationsbereich A erfolgt eine Trennung einströmender Objekte und/oder eine Detektion. Beim dargestellten Beispiel ist im Manipulationsbereich A ein Paar von Ablenkelektroden 76 vorgesehen, die sich im Kanal 71 schräg zur Strömungsrichtung erstrecken. Die Ablenkelektroden umfassen eine untere Elektrode am Kanalboden (nicht dargestellt bzw. verdeckt) und eine obere Elektrode an der oberen Kanalbegrenzung. Zwischen den Elektroden kann das Medium frei durchströmen, wobei jedoch bei Beaufschlagung mit einem hochfrequenten Wechselfeld eine elektrische Feldbarriere aufgebaut wird, die sich im Kanal 71 schräg zur Strömungsrichtung erstreckt.

Die dielektrophoretischen Kräfte sind abhängig vom Objektvolumen. Bei biologischen Objekten werden zur Vermeidung von Membranvorschädigungen Wechselfelder mit Frequenzen oberhalb von 10 MHz verwendet. Beim dargestellten Beispiel erfolgt ein Einströmen eines Zellgemisches bestehend aus großen Zellen 74 und kleinen Zellen 75. Die Spannung zwischen den Ablenkelektroden wird derart gewählt, daß auf die großen Zellen 74 eine genügend große dielektrophoretische Abstoßungskraft wirkt, so daß diese entlang der Erstreckungsrichtung der Ablenkelektroden durch eine Öffnung zwischen den Trennelementen 73b in den zweiten Kanal 72 überführt werden. Die kleinen Zellen 75 hingegen können die Feldbarriere zwischen den Ablenkelektroden 76 passieren und bleiben im ersten Kanal 71.

Stromabwärts in den Kanälen sind Detektoren 78 vorgesehen. Falls an diesen der Vorbeitritt von Zellen z.B. elektrisch oder optisch detektiert wird (z.B. durch eine Widerstandsmessung oder mittels Photodioden), so wird unter Berücksichtigung der Strömungsgeschwindigkeit mit einer bestimmten Verzögerungszeit im Elektroporationsbereich B die Elektroporation mit den Elektroden 77, 77a ausgelöst. Die Porationsparameter und die Elektrodengröße ist dabei an die getrennten Zellgrößen angepaßt. Bei den Elektroden 77, 77a handelt es sich vorzugsweise um dreidimensionale Elektrodenanordnungen, wie sie beispielhaft oben erläutert wurde. Die Elektroden sind wiederum am Boden bzw. Deckel des Kanals 71 oder 72 angebracht. Das Auslösen eines Fusions- oder Porationspulses kann rechnergestützt oder durch einen detektorgekoppelten Schalter erfolgen.

Nach der Permeation können die permeierten Zellen 79, 80 gegebenenfalls dielektrisch gesammelt und fusioniert werden, wobei wiederum Ablenkelektroden benutzt werden.

Das in Fig. 7 besipielhaft gezeigte System läßt sich anwendungsabhängig beliebig auf mehr Kanäle, komplexere Ablenktechniken und Elektroporationen an mehreren Stufen erweitern.

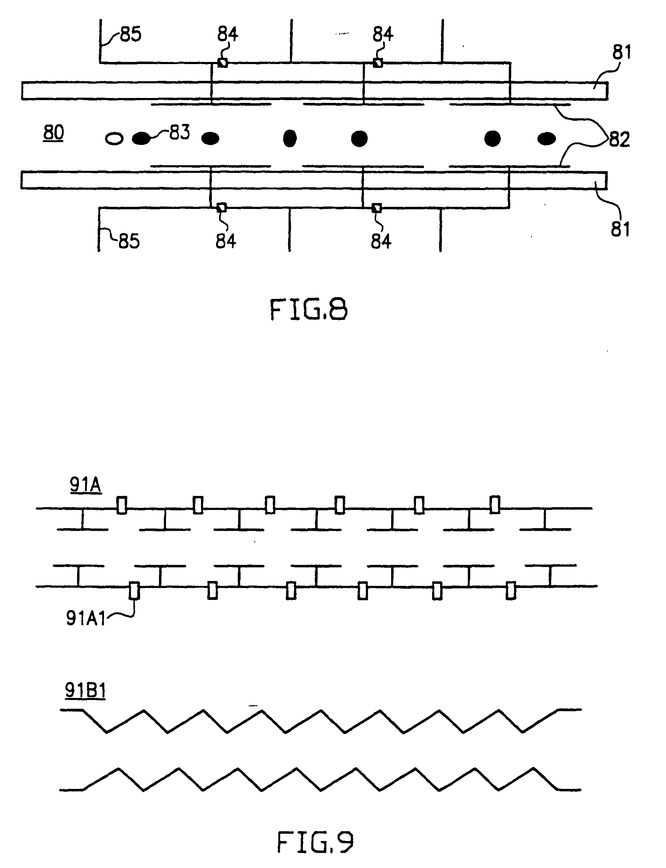

Fig. 8 zeigt ein weiteres Beispiel eines Mikrosystems, das zur mehrstufigen oder kontinuierlichen Permeation ohne zusätzliche Sensoreinrichtungen eingerichtet ist. Beim Mikrosystem gemäß Fig. 8 führt ein Kanal 80 zwischen zwei Begrenzungen 81 eine Strömung eines Mediums mit den Objekten 83. Seitlich an den Kanalbegrenzungen 81 sind Elektroden 82 angebracht, die zur Elektroporation eingerichtet sind. Die Elektroden 82 sind über elektrische Steuerleitungen 85 ansteuerbar, die Schaltelemente 84 zum gezielten Betätigen einzelner Elektroden 82 aufweisen. Die Länge der Einzelelektroden bzw. Elektrodenkombinationen in Kanallängsrichtung, die Strömungsgeschwindigkeit und die gewünschte Porationspulslänge und -wiederholdauer erlauben eine kontinuierliche Permeation der Objekte während des Durchtritts durch den Kanal. Bei Elektroden mit einer Länge von z.B. 1 mm und einer Strömungsgeschwindigkeit von 1 mm/s ist sichergestellt, daß bei einer Pulsperiode von 1 s jede Zelle pro Periode einen Puls erfährt. Der Einsatz einer Vielzahl von Einzelelektroden anstelle einer sich lang erstreckenden, einheitlichen Elektrode besitzt den Vorteil, daß an einer integralen Elektrode Spannungsverluste und Erwärmungen auftreten würden, die die Elektroporation nachteilig beeinflussen könnten.

Der Aufbau der Fokussierungs- und Porationselektroden aus Einzelelektroden gemäß Fig. 8 ermöglicht die Realisierung des folgenden Ansteuerprotokolls. Die Elektroporation basiert auf der Wirkung eines Gleichspannungspulses auf die zu behandelnden Objekte. In der Suspensionslösung ergibt sich während des Gleichspannungspulses an den beteiligten Elektroden eine Ansäuerung bzw. eine Alkalisierung in Elektrodennähe. Um diese pH-Änderungen möglichst gering zu halten, ist erfindungsgemäß vorgesehen, jeweils benachbarte Einzelelektroden mit umgekehrter Feldrichtung zu betreiben, so daß sich Elektroden mit Ansäuerungs- und Alkalisierungserscheinungen abwechseln. Des weiteren kann insbesondere bei einer Anordnung gemäß Fig. 8 vorgesehen sein, daß zunächst ein Einströmen einer Zellgruppe in den Kanal und ein Positionieren der Zellen zwischen den Elektroden erfolgt. Die Zellen werden mit dielektrophoretischen Kräften in Position zwischen den Elektroden gehalten. Anschließend folgt der eigentliche Elektroporationsschritt. Dabei werden aufeinanderfolgend zuerst die am weitesten stromabwärts gelegene Elektrode und anschließend schrittweise jede weitere stromaufwärts gelegene Elektrode mit dem Gleichspannungspuls zur Elektroporation beaufschlagt. Dieses Verfahren hat den Vorteil, daß die Ansäuerungs- und Alkalisierungsprodukte im Suspensionsstrom, der den Kanal durchsetzt, laufend abtransportiert werden, wobei die einzelnen Elektroporationsvorgänge jeweils unter den ursprünglich in der Suspension eingestellten Bedingungen erfolgt.

Fig. 9 zeigt Abwandlungen der Elektrodenanordnung gemäß Fig. 8. Von den dreidimensionalen Elektrodensystemen ist jeweils nur eine Ebene dargestellt. Vorzugsweise wird die zweite Ebene sandwichartig spiegelsymmetrisch über der ersten Ebene angebracht. Die geraden Elektroden 91A parallel zum Kanal besitzen den Vorteil, daß das Feld eine gleichbleibende Feldstärke besitzt. Die Kontakte zu den Subelektroden können über Schaltelemente 91A1 extern gesteuert werden. Alternativ können Kurvenformen 91B (Dreiecksform, Sinusform oder sogenannte "castellated" Form) für bestimmte Zelltypen durch Variation der Feldstärke bevorzugt sein. Segmentierte Einzelelektroden sollten derart dimensioniert sein, daß die Einzelsegmente eine Länge von rd. 100 µm in Kanalrichtung besitzen.

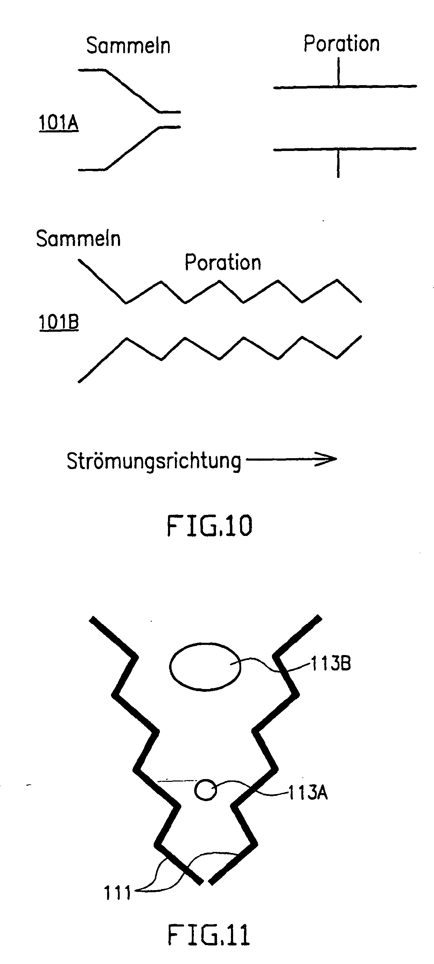

Fig. 10 illustriert die Prinzipien der Kombination von Manipulations- und Elektroporationsbereichen. So können entweder zwei getrennte Strukturen 101A oder eine gemeinsame Struktur 101B benutzt werden. Im Manipulationsbereich ("Sammeln") erfolgt eine Beaufschlagung der Elektroden mit dem Hochfrequenzfeld zur Ausbildung von Feldbarrieren, die die Objekte in den Porationsbereich zusammenführen. Es ist nicht erforderlich, daß das Hochfrequenzfeld für die Dielektrophorese laufend angeschaltet ist. Es ist vielmehr möglich, daß die Dielektrophorese beim Durchtritt eines Objekts aktiviert und dazu zeitlich verzögert (rd. 1 ms) die Elektroporation durchgeführt wird. Die Verzögerung hängt je nach Anwendungsfall von der Strömungsgeschwindigkeit im Kanal ab. Für Puls- bzw. HF-Generatoren für die Elektroporation bzw. die Dielektrophorese ist vorzugsweise eine gemeinsame Massenverbindung vorgesehen.

Eine weitere Abwandlung gegenüber der Anordnung 101B in Fig. 10 ist in Fig. 11 gezeigt. Die Elektrodenstruktur 111 verjüngt sich in Bewegungsrichtung der Teilchen bzw. in Strömungsrichtung im Kanal von einem größeren Abstand der Teilelektroden hin zu einem geringeren Abstand der Teilelektroden. Eine derartige Gestaltung erlaubt es, verschieden große Teilchen an verschiedenen Orten zu behandeln, nämlich das kleinere Teilchen 113A weiter stromabwärts als das größere Teilchen 113B.

Erfindungsgemäß kann eine Elektroporations- oder Fusionsvorrichtung mit einer Einrichtung zur visuellen Beobachtung der Objekte im Kanal bzw. des Ergebnisses der elektrischen Behandlung der Objekte ausgestattet sein. Diese Vorrichtung ist vorzugsweise ein Mikroskop. Ein besonderer Vorteil der Erfindung besteht darin, daß die Anordnung bandförmiger Fokussierungs- und Porationselektroden bei Aufbau des Mikrosytems mit zumindest abschnittsweise transparenten Wänden einen direkten Einblick in den Kanal, insbesondere in den Bereich des Feldminimums zwischen den Elektroden, erlaubt.

## Patentansprüche

1. Vorrichtung zur Elektroporations- und/oder Fusionsbehandlung mikroskopischer Objekte in einem Mikrosystem, die eine Kanalstruktur zur Aufnahme eines Mediums, in dem die Objekte suspendiert sind, und mindestens zwei miniaturisierte Elektroden umfasst, die an den Wänden der Kanalstruktur angeordnet und dazu eingerichtet sind, die Objekte in der Kanalstruktur mit Abstand von deren Wänden und den Elektroden zu halten und mit elektrischen Spannungen zur Elektroporations- und/oder Fusionsbehandlung zu beaufschlagen, wobei die Elektroden Hochfrequenzelektroden zur Positionierung der Objekte in der Kanalstruktur umfassen,
**dadurch gekennzeichnet, dass**
die Elektroden Pulselektroden zur Elektroporation und/oder Fusion der Objekte umfassen.

2. Vorrichtung gemäß Anspruch 1, bei der die Hochfrequenzelektroden und die Pulselektroden durch getrennte Elektrodenelemente oder durch zumindest teilweise verbundene Elektrodenelemente oder gemeinsam durch identische Elektrodenelemente gebildet werden.

3. Vorrichtung gemäß einem der vorhergehenden Ansprüche, bei der die Pulselektroden feldformende Einrichtungen zur Erzeugung eines Porations- und/oder Fusionsmusters aufweisen.

4. Vorrichtung gemäß Anspruch 3, bei der die feldformenden Einrichtungen durch Spitzenelektroden oder Abschirmelemente mit geeigneten Durchtrittsöffnungen gebildet werden, wobei die durch die Spitzenelektroden oder die Abschirmelemente gebildeten wirksamen Elektrodenflächen eine Dimension besitzen, die wesentlich kleiner als eine charakteristische Dimension des jeweils zu behandelnden Objekts ist.

5. Vorrichtung gemäß einem der vorhergehenden Ansprüche, bei der in der Kanalstruktur in Durchflussrichtung des Mediums eine Vielzahl von Pulselektroden angeordnet sind.

6. Vorrichtung gemäß einem der vorhergehenden Ansprüche, bei dem die Kanalstruktur eine Vielzahl von Kanälen jeweils mit miniaturisierten Elektroden umfaßt, die zum gleichzeitigen Durchfluss des Suspensionsmediums mit den zu behandelnden Objekten eingerichtet sind.

7. Elektroporationsgerät, das mit einer Vorrichtung gemäß einem der Ansprüche 1 bis 6 ausgerüstet ist.

8. Elektrofusionsgerät, das mit einer Vorrichtung gemäß einem der Ansprüche 1 bis 6 ausgerüstet ist.

9. Gerät gemäß Anspruch 7 oder 8, das als Mikrosystem mit einer Multielektrodenanordnung gestaltet ist.

10. Verfahren zur Elektroporation und/oder Fusion mikroskopischer Objekte, bei dem die Objekte frei suspendiert mit einem Medium durch eine Kanalstruktur eines Mikrosystems berührungslos mittels negativer Dielektrophorese an mindestens zwei Elektroden zumindest zeitweilig positioniert oder vorbeigeführt werden, die zur Elektroporation und/oder Fusion eingerichtet sind, und die Elektroden mit vorbestimmten Spannungen zur Elektroporation und/oder Fusion der Objekte beaufschlagt werden.

11. Verfahren gemäß Anspruch 10, bei dem die Objekte in der Kanalstruktur mit Hochfrequenzelektroden auf der Grundlage negativer Dielektrophorese in einem sich in Kanallängsrichtung erstreckenden elektrischen Feldminimum positioniert oder geführt werden.

12. Verfahren gemäß Anspruch 10 oder 11, bei dem die Objekte während der Elektroporation bzw. Fusion durch die Kanalstruktur strömen.

13. Verfahren gemäß Anspruch 10 oder 11, bei dem die Objekte während der Elektroporation bzw. Fusion in der Kanalstruktur festgehalten werden.

14. Verfahren gemäß einem der Ansprüche 10 bis 13, bei dem die Objekte mit einem Abstand von den Pulselektroden positioniert oder an diesen vorbeigeführt werden, der gleich einem bis einem halben Durchmesser der Objekte ist.

15. Verfahren gemäß einem der Ansprüche 10 bis 14, bei dem vor der Elektroporation bzw. Fusion eine Erfassung von Objekteigenschaften und/oder eine Trennung von Objekten mit verschiedenen Eigenschaften erfolgt.

16. Verfahren gemäß einem der Ansprüche 10 bis 15, bei dem die Objekte biologische Objekte, insbesondere Zellen, oder synthetische, membranumhüllte Gebilde, insbesondere Liposomen oder Vesikeln, umfassen.

## Claims

1. Apparatus for the electroporation and/or fusion treatment of microscopic objects in a microsystem, which comprises a channel structure to receive a medium, in which the objects are suspended, and at least two miniaturised electrodes, which are arranged on the walls of the channel structure and are fitted to hold the objects in the channel structure at a distance from the walls thereof, and to hold the electrodes and apply electrical voltages for the electroporation and/or fusion treatment thereto, wherein the electrodes comprise high-frequency electrodes for positioning the objects in the channel structure, **characterised in that** the electrodes comprise pulse electrodes for the electroporation and/or fusion of the objects.

2. Apparatus according to Claim 1, wherein the high-frequency electrodes and the pulse electrodes are formed by separate electrode elements or by at least partially connected electrode elements or jointly through identical electrode elements.

3. Apparatus according to one of the preceding claims, wherein the pulse electrodes have field-forming systems for the generation of a poration and/or fusion pattern.

4. Apparatus according to Claim 3, wherein the field-forming systems are formed by pin electrodes or screening elements with suitable passages, wherein the active electrode surfaces formed by the pin electrodes or the screening elements have a dimension that is substantially smaller than the characteristic dimension of the respective object to be treated.

5. Apparatus according to one of the preceding claims, wherein a plurality of pulse electrodes are arranged in the channel structure in the throughflow direction of the medium.

6. Apparatus according to one of the preceding claims, wherein the channel structure comprises a plurality of channels each with miniaturised electrodes, which are arranged for the simultaneous throughflow of the suspension medium with the objects to be treated.

7. Electroporation device, which is equipped with an apparatus according to one of Claims 1 to 6.

8. Electrofusion device, which is equipped with an apparatus according to one of Claims 1 to 6.

9. Device according to Claim 7 or 8, which is configured as a microsystem with a multi-electrode arrangement.

10. Method for the electroporation and/or fusion of microscopic objects, wherein the objects are directed freely suspended with a medium through a channel structure of a microsystem in a non-contact manner by means of negative dielectrophoresis past at least two electrodes or at least intermittently positioned thereon, said electrodes being arranged for electroporation and/or fusion, and predetermined voltages are applied to the electrodes for electroporation and/or fusion of the objects.

11. Method according to Claim 10, wherein the objects are positioned or directed in the channel structure with high-frequency electrodes on the basis of negative dielectrophoresis in an electrical field minimum extending in the longitudinal direction of the channel.

12. Method according to Claim 10 or 11, wherein the objects flow through the channel structure during the electroporation or fusion.

13. Method according to Claim 10 or 11, wherein the objects are held in the channel structure during the electroporation or fusion.

14. Method according to one of Claims 10 to 13, wherein the objects are positioned at a distance from the pulse electrodes or are directed past these at a distance that is equal to one to half a diameter of the objects.

15. Method according to one of Claims 10 to 14, wherein prior to the electroporation or fusion, object properties are determined and/or objects with different properties are separated.

16. Method according to one of Claims 10 to 15, wherein the objects are biological objects, in particular cells, or synthetic structures enclosed by membranes, in particular liposomes or vesicles.

## Revendications

1. Dispositif pour le traitement d'électroperméation et/ou de fusion d'objets microscopiques dans un microsystème, qui comprend une structure du type canal destinée à contenir un milieu dans lequel les objets sont mis en suspension et au moins deux électrodes miniaturisées qui sont disposées au niveau des parois de la structure du type canal et agencées pour maintenir les objets contenus dans la structure du type canal à distance des parois de cette structure et des électrodes et pour les attaquer avec des tensions électriques pour le traitement d'électroperméation et/ou de fusion, les électrodes comprenant des électrodes à haute fréquence destinées à positionner les objets dans la structure du type canal,
**caractérisé en ce que**
les électrodes comprennent des électrodes à impulsions pour l'électroperméation et/ou la fusion des objets.

2. Dispositif selon la revendication 1, dans lequel les électrodes à haute fréquence et les électrodes à impulsions sont constituées par des éléments d'électrodes séparés ou par des éléments d'électrodes au moins partiellement reliés ou encore en commun par des éléments d'électrodes identiques.

3. Dispositif selon une des revendications précédentes, dans lequel les électrodes à impulsions présentent des dispositifs de configuration du champ destinés à produire un dessin de perméation et/ou de fusion.

4. Dispositif selon la revendication 3, dans lequel les dispositifs de configuration du champ sont constitués par des électrodes à pointe ou par des éléments formant écran munis d'ouvertures de passage appropriées, les surfaces d'électrodes effectives constituées par les électrodes à pointe ou par les électrodes formant écran possédant une dimension qui est nettement plus petite qu'une dimension caractéristique de l'objet à traiter.

5. Dispositif selon une des revendications précédentes, dans lequel, dans la structure du type canal, une pluralité d'électrodes à impulsions sont disposées dans la direction de l'écoulement du milieu.

6. Dispositif selon une des revendications précédentes, dans lequel la structure du type canal comprend une pluralité de canaux dont chacun est muni d'électrodes miniaturisées qui sont disposées pour assurer en même temps la circulation du courant du milieu de suspension avec les objets à traiter.

7. Appareil d'électroperméation qui est équipé d'un dispositif selon une des revendications 1 à 6.

8. Appareil d'électrofusion qui est équipé d'un dispositif selon une des revendications 1 à 6.

9. Appareil selon la revendication 7 ou 8, qui a la configuration d'un microsystème ayant une disposition à électrodes multiples.

10. Procédé d'électroperméation et/ou de fusion d'objets microscopiques dans lequel, dans une structure du type canal appartenant à un microsystème, les objets mis en suspension libre avec un milieu sont positionnés au moins temporairement, ou entraînés, au moyen d'une diélectrophorèse négative, au droit d'au moins deux électrodes, qui sont agencées pour l'électroperéation et/ou la fusion, sans entrer en contact avec ces dernières, et les électrodes sont attaquées avec des tensions prédéterminées pour assurer l'électroperméation et/ou la fusion des objets.

11. Procédé selon la revendication 10, dans lequel, dans la structure du type canal, les objets sont positionnés ou entraînés au moyen d'électrodes à haute fréquence sur la base d'une électrophorèse négative, dans un minimum de champ électrique qui s'étend dans la direction longitudinale du canal.

12. Procédé selon la revendication 10 ou 11, dans lequel les objets circulent dans la structure du type canal pendant l'électroperméation ou la fusion.

13. Procédé selon la revendication 10 ou 11, dans lequel les objets sont maintenus immobilisés dans la structure du type canal pendant l'électroperméation ou la fusion.

14. Procédé selon une des revendications 10 à 13, dans lequel les objets sont positionnés ou entraînés au droit des électrodes à impulsions à une distance de ces dernières qui est égale à un diamètre ou jusqu'à un demi-diamètre des objets.

15. Procédé selon une des revendications 10 à 14, dans lequel, avant l'électroperméation ou la fusion, on procède à une acquisition des propriétés des objets et/ou à une séparation d'objets possédant différentes propriétés.

16. Procédé selon une des revendications 10 à 15, dans lequel les objets sont des objets biologiques, en particulier des cellules, ou des complexes synthétiques, entourés d'une membrane, en particulier des liposomes ou de vésicules.
